# EUROPEAN PATENT SPECIFICATION

(11) **EP 2 317 987 B1**
(45) Date of publication and mention of the grant of the patent: **23.04.2014**
(21) Application number: 09780358.9
(22) Date of filing: 09.07.2009
(51) Int. Cl.: A61K 9/20, A61K 9/46, A61K 31/4178, A61K 31/549, A61P 9/04, A61P 9/12, A61P 13/12

(54) **Eprosartan acid compositions**
Eprosartansäure-Zusammensetzungen
Compositions à base d'éprosartan sous forme acide

(30) Priority: 11.07.2008 US 80067; 11.07.2008 EP 08160211
(43) Date of publication of application: 11.05.2011
(73) Proprietor: Abbott Healthcare Products B.V., 1381 CP Weesp (NL)
(72) Inventor: LINK, Paulus A. J., NL-1381 CP Weesp (NL); VAN DER HULST, Marcellus M., NL-1381 CP Weesp (NL); BIELENBERG, Gerhard-Wilhelm, 1381 CP Weesp (DE); VAN DEN AKKER, Cornelis R., NL-1381 CP Weesp (NL)
(74) Representative: Kraak, Hajo
(86) International application number: PCT/EP2009/058724
(87) International publication number: WO 2010/003996

(56) References cited:
- EP-A- 0 955 294
- WO-A-99/25321
- FR-A- 2 882 260
- FR-A- 2 886 150
- US-A- 5 656 650
- US-A1- 2003 133 976
- Excerpt of the European Pharmacopoeia 5.0: 2005
- Committee for medicinal products for human use (EMEA): "Guideline on the investigation of bioequivalence", 2010 pages 1-27,

## Description

### Field of the Invention

The present invention is in the field of treatment of a disorder modulated by blocking angiotensin II (All) receptors, and particularly selected from the group consisting of hypertension, congestive heart failure, renal failure, and combinations thereof, by administering to a subject in need thereof, an eprosartan compound in a dose sufficient to treat such disorders (the Recommended Effective Daily Dose). The invention further relates to providing a drug product that is bio-equivalent with a reference drug product that has eprosartan mesylate as the active substance. Also, the invention pertains to a pharmaceutical dosage unit for the administration of eprosartan and to a pharmaceutical formulation comprising eprosartan.

### Background of the Invention

Eprosartan is (E)-α-[2-n-butyl-1-[(4-carboxy phenyl)methyl]-1H- imidazol-5-yl] methylene-2-thlopheneproplonic acid. Eprosartan is the subject of U.S. Patent No. 5,185,351 (the 351 patent), issued February 9, 1993. See also EP 0 955 294, which has a similar disclosure. EP 0 955 294 describes the synthesis and general use as All receptor antagonists, of eprosartan and related compounds is disclosed. A synthesis example of eprosartan acid is included. The preference in this disclosure is given to the methyl sulfonate (eprosartan mesylate).

The state of the art in respect of the aforementioned method of treatment is the existing commercial form in which eprosartan, namely eprosartan mesylate.

However, the variable and mean absolute bioavailability of eprosartan mesylate is approximately 13%. As a result, high doses may be required for an effective treatment of hypertension, congestive heart failure and renal failure. Effective daily doses range from 400 to 800 mg, calculated on the basis of eprosartan.

The Recommended Effective Daily Dose of eprosartan mesylate is 600 mg, calculated on the basis of eprosartan. In some cases, a starting regimen is prescribed in which an initial dose of 300 mg per day is administered. The Recommended Effective Daily Dose can be achieved by the once daily administration of the aforementioned eprosartan mesylate 600 mg tablets, or by the twice daily administration of eprosartan mesylate 300 mg tablets.

The Recommended Effective Daily Dose can also be characterized with reference to the plasma levels of eprosartan it achieves. For example, following the administration of the eprosartan compound, in the Recommended Effective Daily Dose, to a human subject, the subjects exhibit at least one of:
(a) a mean plasma Cₘₐₓ of eprosartan of between 2200 and 3600 ng/ml; or
(b) a mean plasma AUC₀₋ₜ of eprosartan of between 8000 and 11000 hr·ng/ml.

By way of further background, the following documents reference eprosartan.

US Patent No. 5,656,650 relates to pharmaceutical compositions comprising, in addition to an All receptor antagonist, a second agent being a diuretic, a calcium channel blocker, a β-adrenoceptor blocker, a renin inhibitor, or an angiotensin converting enzyme inhibitor. As to eprosartan, the document exemplifies oral dosage forms of 100 mg eprosartan acid. The total daily dose of the All receptor antagonist is broadly indicated to be from about 5 mg to about 1000 mg. The document does not suggest any change to the aforementioned Recommended Effective Daily Dose.

WO 99/25321 addresses the high tablet weight of eprosartan mesylate dosage units. It is proposed to provide high drug load tablets on the basis of anhydrous eprosartan acid. The document refers to the above-mentioned typical effective dose, viz. 600 mg (calculated based on eprosartan). The document refers to 600 mg dosage units, and does not indicate any alteration of the aforementioned Recommended Effective Daily Dose. The reference apparently does not fully succeed in providing the required 600 mg dosage units. Although a broad dosage range of 50 mg to 1 g is given, the preferred dosage units contain from about 200 to about 400 mg of the eprosartan acid. It is indicated that these are to be taken 1-4 times daily, preferably 1-2 times daily. This is commensurate with the aforementioned state of the art doses of 300 respectively 600 mg.

FR 2 886 150 addresses the administration of active substances the solubility of which varies strongly with varying gastric pH, of which eprosartan is given as an example. It is referred to as a problem in the sense that these drugs, within the same patient, may lead to variable plasma levels depending, e.g., on the time of day the drug is taken, and whether the patient has a fed or empty stomach. The reference proposes to address this problem by providing the active substance with a coating, or by combining it with a matrix, which coating or matrix allows the controlled release of the active substance. As an example, a 300 mg dosage unit of eprosartan is given. The reference does not indicate any alteration of the Recommended Effective Daily Dose i.e., considering the standard dosage amounts in the art, this exemplified dosage unit would normally be given twice a day so as to reach the standard effective dose of 600 mg eprosartan.

FR 2 882 260 refers to multiple daily administrations of All receptor antagonists, on account of their low half-life values. As a solution, it is proposed to provide multi-microparticulate dosage forms for the prolonged release of these compounds. For eprosartan an example is given of a 400 mg microparticulate dosage unit, which is indicated to prolong the duration of release by 6 hours. The document neither indicates the daily doses required, nor does it indicate any alteration of the Recommended Effective Daily Dose.

In respect of the latter two references, it is noted that in the present invention it is particularly desired to provide a straightforward immediate-release formulation for eprosartan.

"Immediate Release Formulation" means any formulation such that by the time eprosartan leaves the stomach, it is either in solution or it is in the form of a suspension of fine particles, i.e. a form from which eprosartan can be readily absorbed. More particularly, the term "Immediate Release" refers to a release of at least 75 %, and preferably at least 90% of the drug in a dissolved form from the dosage form within 90 minutes, preferably within 60 minutes. Most particularly, the term "Immediate Release" refers to a release of at least 75% within 45 minutes. Preferred Immediate Release Formulations release at least 90% of the drug in 30 minutes, more preferably in 15 minutes and most preferably at least 95% of the drug in 15 minutes. The release rates referred to are those as determined in accordance with The United States Pharmacopeia (USP) as described in Example 8.

None of the foregoing references addresses the desire to provide an eprosartan dosage unit on the basis of an eprosartan drug substance that has a better bioavailability than the standard in the art, viz. eprosartan mesylate.

### Summary of the Invention

It would be desired to provide an eprosartan compound having a better bioavailability than the current standard, eprosartan mesylate. This would allow achieving the Recommended Effective Daily Dose on the basis of a lower amount of drug or, in other words, providing bio-equivalent tablets, having a lower amount of drug. This is generally recognized as a benefit to patients.

Surprisingly, it was found that eprosartan acid is such a better bioavailable eprosartan compound.

Thus, the invention, in one aspect, is eprosartan acid for use in a treatment as defined in claim 1, wherein the daily dose of eprosartan acid is 440-460 mg.

In another aspect, the invention presents an immediate release pharmaceutical formulation comprising of eprosartan acid 440-460 mg, and particularly of about 450 mg.

The invention enables the use of eprosartan acid for the purpose of providing a drug product that is bio-equivalent with a reference drug product comprising eprosartan mesylate as the active substance, wherein the bio-equivalent dose of eprosartan acid is lower than the reference dose of eprosartan mesylate, calculated on the basis of eprosartan acid.

The invention further relates to formulations of eprosartan having improved bioavailability compared with the present marketed eprosartan mesylate formulations, processes for manufacturing these formulations and methods of using the eprosartan formulations of the present invention in the treatment of certain disease states in mammals, in particular man.

### Description of the Figures

Figure 1 depicts the XRPD pattern of polymorphic form α eprosartan acid
Figure 2 depicts the XRPD pattern of polymorphic form β of eprosartan acid
Figure 3 is a graph representing Geometric Mean Eprosartan Plasma Concentration Time Profiles for all Treatments discussed in Example 8 (0-10 hour profiles)

### Detailed Description of the Invention

### 1. Effective dose in the treatment of All receptor-modulated disorders

Eprosartan serves in the treatment of disorders modulated by blocking angiotensin II (All) receptors. The invention particularly pertains to disorders selected from the group consisting of hypertension, congestive heart failure, renal failure, and combinations thereof.

The method of treatment to which the invention is applied, is by administering to a subject in need thereof, a dose of 440-460 mg of eprosartan acid.

As mentioned above, the Recommended Effective Daily Dose is 600 mg (calculated based on eprosartan) as present in eprosartan mesylate.

The Recommended Effective Daily Dose effective dose serves to introduce into the subject's plasma a level of eprosartan corresponding to the level introduced upon the administration of crystalline eprosartan mesylate in a dosage amount of 600 mg.

More particularly, the Recommended Effective Daily Dose is a dose of an eprosartan compound following its administration to human subjects, and the subjects exhibit at least one of:
(a) A mean plasma Cₘₐₓ of eprosartan of between 2200 and 3600 ng/ml;
(b) A mean plasma AUC₀₋ₜ of eprosartan of between 8000 and 11000 hr·ng/ml;
"AUC" means the Area Under plasma concentration time Curve.

According to the invention, eprosartan acid is administered in an effective daily dose of 440-460 mg. Most preferably, eprosartan is provided in a daily dose of 450 mg.

The invention also pertains to dosage units comprising eprosartan acid in a single daily dose within the foregoing range, and most preferably comprising 450 mg of eprosartan acid.

The invention has for its purpose to provide a treatment that can be considered similar to the treatment with eprosartan mesylate. By "similar" it is meant that the treatment is with an eprosartan compound at a dosage level considered bio-equivalent with the appropriate dosage level of eprosartan mesylate.

The substantial reduction of the eprosartan strength in the treatment of the invention is unforeseen in the art. Therein, the problem of finding a better bioavailable, bio-equivalent alternative to eprosartan mesylate is not addressed. References on eprosartan acid, and salts, include a variety of patent documents from the early 1990s in which formulation examples are included, e.g. of eprosartan acid 75mg or 100mg: EP 0 955 294, WO 92/10189, US 5,418,250, US 5,185,351, WO 199/10097, US 5,656,650, WO 92/10181, US 6,034,114, WO 92/10182, US 6,028,091, EP 561 977, US 6,025,380, EP 561 876. These examples are not related to a method of treatment using eprosartan at the aforementioned effective doses.

Whilst the aforementioned references thus do not address the present invention, the formulations of eprosartan acid disclosed therein could in fact be put to use in the present invention, provided that they are administered at the above-discussed appropriate lower dosage level as compared to the dosage level of eprosartan mesylate.

### 2. Eprosartan drug product for use in the treatment of All receptor-modulated disorders

To the extent that the invention is in the field of a method of treatment of the human body, it can be viewed as residing in an eprosartan drug product for use in such a method of treatment.

Thus, the invention pertains to an eprosartan drug product for use in a method of treatment of a disorder modulated by blocking angiotensin II (All) receptors, and particularly selected from the group consisting of hypertension, congestive heart failure, renal failure, and combinations thereof, ,by administering to a subject in need thereof, a daily dose of 440-460 mg of eprosartan acid in a single immediate release oral formation.

### 3. Providing a bio-equivalent eprosartan drug product

By virtue of this invention, it has become possible, and unexpectedly so, to provide a drug product that is bio-equivalent with the existing eprosartan mesylate drug product, yet on the basis of a lower dosage strength of eprosartan.

The invention thus enables the use of eprosartan acid for the purpose of providing a drug product that is bio-equivalent with a reference drug product comprising crystalline eprosartan mesylate as the active substance, wherein the bio-equivalent dose of eprosartan acid is lower than the reference dose of eprosartan mesylate, calculated on the basis of eprosartan acid.

The invention enables providing an eprosartan drug compound that is bio-equivalent with a reference drug product comprising eprosartan mesylate, by providing the eprosartan drug compound and registering it with a regional or national authority responsible for the grant of a Marketing Authorization for drugs (such as the US FDA (United States Food and Drug Administration)) on the basis of the appropriate bio-equivalency studies, wherein the eprosartan drug compound is 440-460 mg eprosartan acid. The bio-equivalent dose of eprosartan acid is lower than the reference dose of eprosartan mesylate, calculated on the basis of eprosartan acid. The concept of bio-equivalency is known in the art.

In the framework of the present invention the expression bioequivalent means: the absence of a significant difference in the rate and extent to which the eprosartan becomes available after administration of the new formulation and the 600 mg mesylate formulation in plasma under similar conditions in an appropriately designed study. The absence of a "significant difference" as mentioned above in the framework of the present invention means that the 90% confidence interval for the AUC ratio and the 90% confidence interval for the Cₘₐₓ ratio are lying within an acceptance interval of 80-125%, preferably in an acceptance interval of 90-111 % and most preferably in an acceptance interval of 95-105%. Appropriate designs for bio-equivalence studies are well known to the skilled person.

This concept of bio-equivalency serves to avoid an undue repetition of human clinical trials, by allowing a drug manufacturer (usually indicated as a "generic drug manufacturer") to register a copy of a reference drug on the basis of limited trials, in which it is merely shown that the copy is bio-equivalent with the reference drug (the inference being that a bio-equivalent drug by definition will have similar efficacy and safety).

The result of the invention, in terms of providing a drug that is bio- equivalent with eprosartan mesylate, is that the effective daily dose of eprosartan acid will be lower than the Recommended Effective Daily Dose, calculated on the basis of eprosartan acid, as present in eprosartan mesylate, the reference drug.

Without wishing to be bound by theory, the present inventors believe that a further advantage can be attributed to the relatively high bioavailability of eprosartan acid, viz. a lower variability of pharmacokinetic parameters. Thus, the selection of eprosartan acid as the active ingredient will lead to better possibilities of providing a bio-equivalent product, with a lower sensitivity for small dosage or formulation changes.

### 4. Eprosartan acid

(E)-α-[2-n-Butyl-1-[(4-carboxyphenyl)methyl]-1H-imidazol-5-yl]methylene-2-thiophenepropionic acid (further referred to as eprosartan acid) is known to exist in a crystalline form from US 5,185,351. Reference should be made to US 5,185,351 for its full disclosure. Eprosartan acid is an amphiphilic molecule containing two acidic (allylic carboxylic acid and phenylic carboxylic acid) and one basic (imidazole) functional groups. At lower pH (below 2) the imidazole nitrogen will be protonated (form ii). As the pH increases, the allylic carboxylic group will be deprotonated (form iii). Estimated pKa of the allylic carboxylic group is 2.9. As the pH increases further, the phenylic carboxylic group will be deprotonated (form iv) followed by the deprotonation of the protonated imidazole group (form v). The estimated pKa of the phenylic carboxylic group is 5.9 and that of imidazole group is 6.8.

In accordance with the invention, the eprosartan acid is not restricted to any particular form, e.g., crystalline or amorphous. Preferably, the eprosartan acid is crystalline.

Crystalline eprosartan acid exists in two different polymorphic forms, further mentioned as the (alpha) and β (beta) forms. The polymorphic form α (alpha) of eprosartan acid has the highest thermo-dynamic stability up to 200°C. Although the present invention is illustrated by using the α (alpha) form of eprosartan acid, the invention can be used in the same way by using the β (beta) form of eprosartan acid

The α polymorphic form of eprosartan acid is prepared by crystallizing the compound from acid medium (e.g. from acetic acid or formic acid) or by crystallizing the compound from ethanol/water while seeding. This polymorphic form is also described in WO 99/25321 and US 5,185,351, although these publications are silent as to the way of preparation. The β polymorphic form of eprosartan acid is prepared by crystallizing the compound from ethanol/water without seeding.

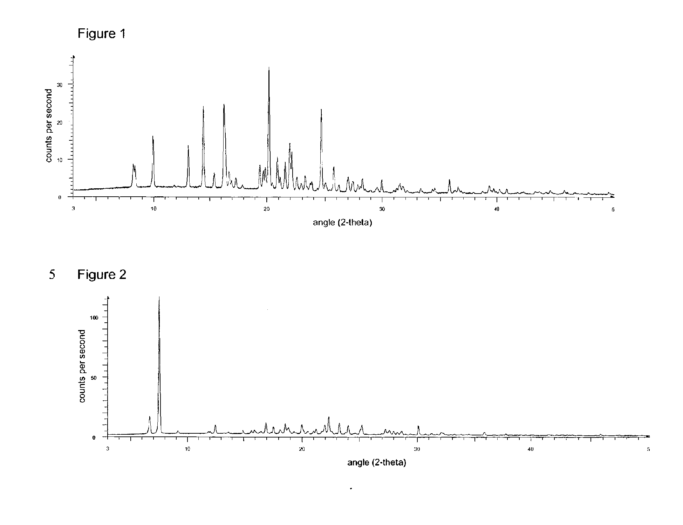

The anhydrous forms of eprosartan acid polymorphic form β exhibits a melting range, expressed as onset value in the DSC curve, of 264-269°C. The anhydrous forms of eprosartan acid polymorphic form α exhibits a single thermal event, a melting endotherm at about 269 °C. No significant weight loss prior to melting is observed in its TGA (thermo gravimetric analysis), for both compounds, suggesting that these compounds do not contain significant quantities of surface adsorbed water and/or residual solvents. The powder X-ray diffraction (XRD) patterns of eprosartan acid polymorphic form α, is presented in Figure 1. The powder X-ray diffraction (XRD) patterns of eprosartan acid polymorphic form β, is presented in Figure 2.

### 5. Formulations

The present invention provides a formulation comprising eprosartan acid containing an amount of active ingredient that is considerably lower than a comparable formulation containing eprosartan mesylate while the same bioavailability of the active ingredient is maintained. More specifically, the present invention provides low dose eprosartan formulation comprising an amount of eprosartan acid, which is between 68.3% and 81.7%, particularly between 70% and 80%, and more particularly 75%, of the calculated amount eprosartan acid present in the comparable formulation comprising eprosartan mesylate. This means, e.g., that an eprosartan formulation comprising preferably about 450 mg, is bioequivalent to a comparable formulation containing 600 mg eprosartan acid in the form of eprosartan mesylate. This finding is surprising and unknown in the art.

A comparable formulation in the framework of the present invention means a formulation having the same release characteristics, but for the amount of the active ingredient, i.e., as is known to the skilled person, the rate and amount of release of an active compound from a pharmaceutical formulation can be influenced by the composition of excipients (i.e. the non-active constituents) of the formulation.

The present invention enables providing a pharmaceutical composition wherein following administration of the formulation comprising an amount of eprosartan acid which is between 70% and 80%, and more particularly 75%, of the calculated amount of eprosartan acid present in the comparable formulation comprising eprosartan mesylate and following administration of the both formulations to human subjects, the subjects exhibit at least one of:
(a) A mean plasma Cₘₐₓ ratio between 0.8-1.25 when comparing the eprosartan acid formulation with a comparable eprosartan mesylate formulation.
(b) A mean plasma AUC₀₋ₜ ratio between 0.8-1.25 when comparing the eprosartan acid formulation with a comparable eprosartan mesylate formulation.

The indicated plasma Cₘₐₓ and plasma AUC₀₋ₜ ratios are preferably between 0.9-1.11 and even more preferably between 0.95-1.05.

Another aspect of the invention provides oral solid dosage forms of eprosartan acid according to the present invention, for the treatment of diseases in which blockade of angiotensin II receptors is indicated, for example, in the treatment of hypertension, congestive heart failure and renal failure.

The formulations containing eprosartan acid exhibit significantly higher solubilities and faster dissolution in water, as well as in gastrointestinal fluids, than the comparable formulation containing eprosartan mesylate. The relative bioavailability in dogs for the eprosartan acid (administered as polymorphic form α) formulation (which formulation is comparable in the composition and amount of auxiliary substances) is 61 % higher and the mean AUC_{0→t} and Cmax values increased 11% and 23%, respectively, when compared to the commercial eprosartan mesylate formulation. Consequently, lower strength tablets are needed for effective treatment of hypertension, congestive heart failure and renal failure, resulting in lower cost of goods and hence, significantly improved patient compliance.

The present invention therefore relates to a low dose eprosartan formulation containing 440-460 mg, and preferably about 450 mg of eprosartan acid, being bioequivalent to the same formulation containing 735 mg crystalline eprosartan mesylate. The eprosartan acid in the abovementioned formulation is preferably in the crystalline form, the amount of eprosartan in the formulation is preferably higher than 30 % w/w and lower than 70% w/w. Further, the formulation does not contain more than 5% w/w of arginine and preferably no arginine at all. In respect of bioequivalence with the above-discussed 600 mg eprosartan formulation (735 mg of eprosartan mesylate), in a particularly preferred embodiment the daily dose applied in the invention, as preferably comprised in a single daily dosage unit, an amount of eprosartan acid of 450 mg.

In accordance with the present invention, it has been found that stable tablet formulations containing (E)- a -[2-n-butyl-1-[(4-carboxyphenyl)methyl]-1H-imidazol-5-yl]methylene-2-thiophenepropionic acid can be produced by pharmaceutical processes known in the field, e.g., wet-granulating, direct compression, spray drying, slugging etc.

In a further, more specific, embodiment the formulation of the present invention comprises (E)-α-[2-n-butyl-1-[(4-carboxyphenyl)methyl]- H-imidazol-5-yl]methylene-2-thiophenepropionic acid and an alkali system in an amount greater than 20% w/w of the composition, preferably comprising a mixture of at least two alkaline compounds in the ratio 1:20 to 20:1 and optionally one or more pharmaceutically acceptable excipients.

In further preferred embodiment, the alkali system comprises a mixture of sodium bicarbonate and sodium carbonate such as Buffered Soda^{™} (mixture of 41.5% - 44.5% w/w sodium carbonate and 58.5% - 55.5% sodium bicarbonate) and Effer-Soda™-12 (mixture of 83-90% w/w sodium bicarbonate and 10-17% w/w sodium carbonate), marketed by SPI Pharma. Effer-Soda™-12 is a highly stable, surface modified sodium bicarbonate powder. It is produced by converting the surface of sodium bicarbonate particles to sodium carbonate. Primarily, Effer-Soda™-12 contains 83-90% w/w sodium bicarbonate and 10-17% w/w sodium carbonate. The outer layer of sodium carbonate absorbs moisture (from the atmosphere or composition) and forms sodium sesquicarbonate, which is stable up to 70°C temperature. This protection mechanism provided by the heat stable sodium sesquicarbonate prevents early effervescent reaction at ambient and elevated temperature storage conditions.

Surprisingly, it has been found that using an alkaline compound in the formulation, alone or in a mixture, e.g. Effer-Soda™-12, the bioavailability of eprosartan acid as evidenced during an *in vivo* dog study is even more increased compared with eprosartan acid in the existing commercial formulation. The mean relative bioavailability in dogs (measured in 6 dogs) for the eprosartan acid (administered as polymorphic form α) formulation containing Effer-Soda™-12 is 107 % higher and the mean AUC_{0→t} and Cmax values increased 31% and 30%, respectively, when compared to the commercial eprosartan mesylate formulation. Consequently, lower strength tablets are needed for effective treatment of hypertension, congestive heart failure and renal failure, resulting in lower cost of goods and hence, significantly improved patient compliance.

In yet another embodiment, for ease of providing an eprosartan acid dosage unit that is bioequivalent with 600 mg of eprosartan mesylate, the formulation is selected so as to provide a plasma concentration time curve of similar shape to that of the reference eprosartan mesylate formulation. To this end, the eprosartan acid is preferably formulated with lactose as an excipient, more preferably lactose monohydrate 200M. Such a preferred lactose grade is marketed e.g. as Pharmatose ® 200M. Other suitable excipients include other grades of alpha lactose monohydrate, dextrose, fructose, sucrose, cellulose, and polyalcohols. Polyalcohols, preferably spray-dried polyalcohols, include mannitol, sorbitol, and xylitol.

In connection herewith, the invention also pertains to a pharmaceutical formulation comprising the aforementioned effective daily dose of eprosartan, and at least one excipient selected from the group consisting of alpha lactose monohydrate and polyalcohols, more preferably lactose monohydrate 200M. Preferred formulations further comprise crospovidone (cross-linked N-vinyl-2-pyrrolidone) as a disintegrant. A still more preferred formulation comprises granules of lactose monohydrate 200M and microcrystalline cellulose, more preferably silicified microcrystalline cellulose as a binder, starch and crospovidone. As extragranular components preferably also crospovidone is present, as well as magnesium stearate (lubricant).

A most preferred dosage unit comprises: 450 mg of eprosartan acid, 71.25 mg of alpha lactose monohydrate 200M (particularly Pharmatose® 200M), 60.0 mg of silicified microcrystalline cellulose (particularly Prosolv® SMCC90), 9.040 mg of starch, and 7.5 mg of crospovidone (particularly Polyplasdone® XL10. Preferably, the extragranular components are 7.5 mg of crospovidone (which brings the total of crospovidone in the dosage unit to 15 mg) and 7.5 mg of magnesium stearate.

The formulations of the invention may comprise a diuretic compound as a further active ingredient, such as hydrochlorothiazide or furosemide, the diuretic compound preferably being hydrochlorothiazide. The amount of the diuretic present in a dosage unit is from about 1 mg to about 500 mg, preferably between 10 and 200 mg. The most preferred dose for hydrochlorothiazide is 12.5 mg. Naturally, these dose ranges can be adjusted on a unit basis as necessary to permit divided daily dose and, as noted above, the dose will vary depending on the nature and severity of the disease, weight of patient, special diets and other factors.

### 6. Release

The formulation according to the present invention may be produced as an immediate release oral solid dosage form (capsule or tablet). As used herein, granulation means a solid containing the drug substance mixed with pharmaceutically acceptable carriers or excipients.

As mentioned above, the present invention pertains to dosage forms of the immediate release type. This refers to a release of at least 75 %, and preferably at least 90% of the drug in a dissolved form from the dosage form within 90 minutes, preferably within 60 minutes. Preferred immediate release formulations release at least 90% of the drug in 30 minutes, more preferably in 15 minutes and most preferably at least 95% of the drug in 15 minutes. The release rates referred to are those as determined in accordance with The United States Pharmacopeia (USP).

In a particularly preferred embodiment, in all aspects of the invention, the eprosartan formulation used is one exhibiting a release profile of: 36% in 5 minutes, 95% in 15 minutes, and 100% in 30 minutes.

The release is determined in accordance with USP. In particular, this refers to dissolution testing using the USP Dissolution Apparatus II with a dissolution medium 0.2 M phosphate buffer at pH 7.5, a medium volume of 1000ml with a temperature of 37 ± 0.5°C at a paddle speed of 50 rpm, taking samples with a sample volume of 10 ml and measuring in a QS Flow cell with a path length of 1 mm at a wavelength of 235 nm. The 0.2 M phosphate buffer was prepared by dissolving 302.6 g of disodium hydrogen phosphate dihydrate and 40.8 g of potassium dihydrogen phosphate in 10 litres of pure $ water. The pH was adjusted to 7.50 ± 0.05 with addition of either 5 M sodium hydroxide or 85% phophoric acid.

### 7. Manufacture

Eprosartan acid can be manufactured in accordance with the aforementioned '351 patent. It is preferably purified by recrystallization.

Pharmaceutical formulations of eprosartan acid can be manufactured generally in accordance with techniques known in the art. Preferably, the formulations are made in a wet-granulation process comprising dry mixing the active ingredient and excipients, adding water, and executing one or more granulation steps, e.g. in a fluid bed granulator and processing the resulting granules into a dosage form, such as filling into capsules, or compressing into tablets. Carriers or excipients may include e.g. diluents, binders, and disintegrants. Further, particularly before tableting, a lubricant such as magnesium stearate can be added. Preferred diluents are lactose, microcrystalline cellulose, starch; the latter can also serve as a disintegrant. The carriers or excipients commonly used in pharmaceutical industry are well described in the literature, e.g. the Handbook of Pharmaceutical Excipients, A. Wade and P. J. Weller (Editors), American Pharmaceutical Association (1994). The dosage forms can be capsules or tablets for immediate release.

Any combination of pharmaceutically acceptable carriers or excipients, e. g. diluents, fillers, binders and disintegrants, in desired proportions may be utilized with the spray dried or fluid bed granulated drug substance and immediate release dosage forms of the present invention.

Pharmaceutically acceptable crystallization inhibitors include poly (vinyl pyrrolidone) and urea. Fillers and diluents include, but are not limited to, the following: lactose (hydrous as well as anhydrous), starch (unmodified (corn starch) or modified (for example, Starch 1500 available from Colorcon)), mannitol, sorbitol, cellulose, inorganic sulfates and phosphates. Disintegrants include, but are not limited to, the following: sodium starch glycolate, sodium carmellose and crosslinked polyvinyl pyrrolidone, and binders include, but are not limited to, the following: gelatin, corn starch, modified starch (Starch 1551, pregelatinized starch), hydroxypropyl methyl cellulose (HPMC), hydroxypropyl cellulose (HPC), sodium carboxy methyl cellulose, alginic acid, acacia and amino acids such as glycine, L-arginine, etc. Examples of excipients suitable for modified release applications include, but are not limited to, the following: high molecular weight HPMCs, polymethacrylate polymers known as Eudragit, polyethylene oxide, Polyox ® (Union Carbide Corporation), modified ethyl cellulose, Surelease® (Colorcon), crosslinked acrylic acid polymers, Carbopol® (BF Goodrich Speciality Chemicals) and waxy materials, such as glyceryl behenate (Compritol®), glyceryl palmitostearate (Precirol®), and Gelucires® [all from Gattefosse s. a., France] and carnauba wax.

Preferably, the pharmaceutically acceptable excipients used as bulking agents during the spray drying/granulation process of this invention are lactose, mannitol, Povidone (PVP), sucrose, sodium starch glycolate, and microcrystalline cellulose to be incorporated in stable oral solid dosage forms of eprosartan by blending with additional excipients in desired proportions. More preferably, the excipients used as bulking agents during the spray drying/granulation process are mannitol/lactose, microcrystalline cellulose, sucrose, sodium starch glycolate and Povidone (PVP). Most preferably, the excipients used as bulking agents during the spray drying/granulation process are lactose, microcrystalline cellulose and sodium carmellose.

Preferably, the bulking agents used in the formulation are present in 2-80% on a weight for weight basis. Most preferably, the bulking agent(s) may be present at as low as 5-50% on a weight for weight basis.

The process for preparing the solid dosage forms in accordance with the present invention may be carried out using a combination of a blender/stirrer, a spray dryer or a fluid bed granulator, a comminuting mill, sieving equipment, a powder blender, a capsule filling machine or a tableting machine. Optionally, the spray-dried material may be processed using a rotogranulator to produce spherical granules, which may be polymer film coated to impart modified release properties.

Thus, the present invention provides a pharmaceutical composition, which comprises (E)-α-[2-n-butyl-1-[(4-carboxyphenyl)methyl]-1H-imidazol-5-yl]methylene-2-thiophenepropionic acid. The pharmaceutical composition is adapted for oral administration. The composition is presented as a unit dose pharmaceutical composition containing from 440 to 460 mg of (E)-α-[2-n-butyl-1-[(4-carboxyphenyl)methyl]-1H-imidazol-5-yl]methylene-2-thiophenepropionic acid, preferably 450 mg. Such a composition is normally taken one time daily. The preferred unit dosage forms include tablets or capsules. The compositions of this invention may be formulated by conventional methods of admixture such as blending, filling and compressing. Suitable pharmaceutically acceptable excipients for use in this invention include diluents, fillers, binders and disintegrants. Preferably, the composition is a dry-mix formulation as described above.

(E)-α-[2-n-Butyl-1-[(4-carboxyphenyl)methyl)-1H-imidazol-5-yl]methylene-2-thio-phenepropionic acid may be co-administered with other pharmaceutically active compounds, for example, in physical combination or by sequential administration. Conveniently, the compound of this invention and the other active compound are formulated in a pharmaceutical composition. Thus, this invention also relates to pharmaceutical compositions comprising (E)-α-[2-n-butyl-1-[(4-carboxyphenyl)methyl]-1H-imidazol-5-yl]- methylene-2-thiophenepropionic acid, a pharmaceutically acceptable carrier, and a second pharmaceutically active compound selected from the group consisting of a diuretic, a calcium channel blocker, a β-adrenoceptor blocker, a renin inhibitor, and an angiotensin converting enzyme inhibitor. Examples of compounds which may be included in pharmaceutical compositions in combination with (E)-α-[2-n-butyl-1-[(4-carboxyphenyl)methyl]-1H-imidazol-5-yl]methylene-2-thiophenepropionic acid are diuretics, particularly a thiazide diuretic, such as hydrochlorothiazide, or a loop diuretic, such as furosemide, calcium channel blockers, particularly dihydropyridine antagonists, such as nifedipine, β-adrenoceptor blockers, such as propranolol, renin inhibitors, such as enalkinen, and angiotensin converting enzyme inhibitors, such as captopril or enalapril.

No unacceptable toxicological effects are expected when (E) - α - [2-n-butyl-1-[(4-carboxyphenyl)methyl]-1H-imidazol-5-yl]methylene-2-thiophenepropionic acid is administered in accordance with the present invention.

(E)-α-[2-n-Butyl-1-[(4-carboxyphenyl)methyl]-1H-imidazol-5-yl]methylene-2-thio-phenepropionic acid is useful for treating diseases in which blockade of the angiotensin II receptor would be beneficial. Preferably, this compound is used alone or in combination with said second pharmaceutically active compounds in the treatment of hypertension, congestive heart failure and renal failure. Additionally, (E)-α-[2-n-butyl-1-[(4-carboxy-phenyl)methyl]-1H-imidazol-5-yl]methylene-2-thiophenepropionic acid is of value in the treatment of left ventricular hypertrophy regression, diabetic nephropathy, diabetic retinopathy, muscular degeneration, hemorrhagic stroke, primary and secondary prevention of infarction, prevention of atheroma progression and the regression of atheroma, prevention of restinosis after angioplasty or bypass surgery, improving cognitive function, angina, glaucoma, and CNS disorders, such as anxiety.

The following examples are illustrative of the instant invention. These examples are not intended to limit the scope of this invention as defined hereinabove and as claimed herein below.

### Example 1a. Analytical methods.

XRPD patterns were measured on a diffractometer using monochromatic CuKα radiation (tube voltage 40 kV, tube current 40 mA) at room temperature. IR spectra were recorded on a Fourier transform IR spectrometer in attenuated total reflectance (silicon crystal) with a spectral resolution of 2 cm⁻¹ using a mercury cadmium telluride detector. Melting points were determined on a DSC apparatus as onset temperatures of the melting endotherm using 40 µL aluminum crucibles with a pierced lid. Temperature program: heating from 25 °C up to 300 °C with 10 K min⁻¹. N₂ atmosphere at a flow of 80 mL min⁻¹.

### Example 1b. Materials.

Eprosartan acid can be prepared as described in US 5,185,351. The formulation indicated as "commercial tablet" containing eprosartan mesylate in an amount corresponding with 600 mg eprosartan is commercially avaible and contains apart from the active ingredient microcrystalline cellulose, lactose monydrate, pregelatinised starch, crospovidone and magnesium stearate in the core and Opadry White, Hypromellose, Macrogol 400, Polysorbate 80 and Titanium dioxide in the coating.

### Example 2. Preparation of polymorphic form α of eprosartan acid.

### Example 2.1 Preparation of polymorphic form a of eprosartan acid from acetic acid

Eprosartan acid (50 g) was dissolved in 125 ml acetic acid by heating to 110°C. The solution was cooled to 20°C in about 1 hour and aged at this temperature for 1 hour. The product was isolated by filtration, washed twice with 50 ml water and successively dried at 65°C under vacuum to give 41 g eprosartan in the α (alpha) polymorph. The XRPD pattern of polymorphic form α is given in Figure 1.

### Example 2.2 Preparation of polymorphic form α of eprosartan acid from acetic acid/methanol

Eprosartan acid (100 g) was dissolved in 200 ml acetic acid by heating to 110°C. The solution was cooled to 10°C in about 1 hour; during this cooling, starting from 70°C 200 ml methanol was added slowly. The crystallization started at 52°C when approximately half of the methanol was added. The resulting slurry was aged at 10°C for 1 hour. The product was isolated by filtration, washed twice with 100 ml methanol and successively dried at 65°C under vacuum to give 93 g eprosartan in the α (alpha) polymorph.

This procedure can also be carried out using ethyl acetate, isopropanol, ethanol, acetone, acetonitrile or water instead of methanol.

### Example 2.3 Preparation of polymorphic form α of eprosartan acid from formic acid/water

Eprosartan (50 g) was dissolved in 75 ml formic acid by heating to 50°C. At this temperature 200 ml water was added in about 40 minutes to crystallize the product. The resulting slurry was cooled to 15°C in about 30 minutes and aged at this temperature for 1 hour. The product was isolated by filtration, washed twice with 50 ml water and successively dried at 65°C under vacuum to give 45 g eprosartan in the α (alpha) polymorph.

### Example 2.4 Preparation of polymorphic form α of eprosartan acid from ethanol/water by seeding

A slurry of eprosartan (106.8 g water wet, containing approximately 100 g eprosartan) in 125 ml ethanol and 73 ml water was heated to 55°C, while 61.8 g of an aqueous 32% sodium hydroxide solution was slowly added, to give a clear yellow solution having a pH of 12.9.

At 55°C the solution was acidified with 32% hydrochloric acid until pH 6.4. At this pH the crystallization was induced by seeding with 2 g eprosartan having the alpha polymorph. After aging the crystal slurry for 30 minutes, the acidification was continued to until a final pH of 5.2.

The slurry was cooled to 20°C before the product was isolated by filtration. The product was washed twice with 100 ml of a 1:1 mixture of ethanol and water and dried at 65°C under vacuum to give 97 g eprosartan in the α (alpha) polymorph.

### Example 3. Preparation of polymorphic form β of eprosartan acid

A slurry of eprosartan (100 g) in 125 ml ethanol and 75 ml water was heated to 55°C, while 62.6 g of an aqueous 32% sodium hydroxide solution was slowly added, to give a clear yellow solution having a pH of 12.8.

At 55°C the solution was acidified with 37% hydrochloric acid until pH 5.2 to crystallize the product.

The slurry was cooled to 20°C and aged at this temperature for 1 hour before the product was isolated by filtration. The product was washed 3 times with 100 ml of a 1:1 mixture of ethanol and water and dried at 65°C under vacuum to give 97 g eprosartan in the beta polymorph. The XRPD pattern of polymorphic form beta is given in Figure 2.

### Example 4. Rearrangement of polymorphic form β into polymorphic form α of eprosartan acid.

Mixtures of α (alpha) and β (beta) polymorphs of eprosartan (6 g each) were stirred at 60°C under the following conditions:
a) The eprosartan mixture was suspended in 15 ml ethanol, 18 ml water, 3.47 g NaCl and 0.56 ml 37% hydrochloric acid.
b) The eprosartan mixture was suspended in 15 ml ethanol, 18 ml water.
c) The eprosartan mixture was suspended in 15 ml formic acid and 40 ml water.

The mixtures were sampled at regular intervals. The sample was filtered to isolate the eprosartan, which was then washed with water and dried before analysis on polymorphism. The samples obtained from condition a) and c) showed full conversion into the alpha polymorph after 6 hours. The sample obtained from condition b) showed full conversion into the alpha polymorph after 1 week.

### Example 5. Preparation of a formulation containing 420 mg eprosartan acid by wet granulation [Reference Example]

A formulation is prepared having the composition listed in Table 1.

**Table 1**

| Component | %w/w | mg/700 mg* | Function | Reference to Standard |
|---|---|---|---|---|
| Intra-granular Components | | | | |
| Eprosartan acid | 60.0 | 420.0 | Active | Internal Monograph |
| Pharmatose 200M | 19.0 | 133.0 | Diluent | Ph.Eur. |
| Avicel PH102 | 8.0 | 56.0 | Compression Aid | Ph.Eur. |
| Starch 1500 | 8.0 | 56.0 | Binder | Ph.Eur. |
| Ac-Di-Sol | 3.0 | 21.0 | Disinteprant | Ph.Eur. |

| Extra-granular Components | | | | |
|---|---|---|---|---|
| Ac-Di-Sol | 1.0 | 7.0 | Disintegrant | Ph.Eur. |
| Magnesium Stearate | 1.0 | 7.0 | Lubricant | Ph.Eur. |

| Film Coating | | | | |
|---|---|---|---|---|
| Opadry Yellow 03B22291 | 4.0 | 28.0 | Coating | Internal Monograph |
| Total Tablet weight | | 728 mg | | |

| | | | | |
|---|---|---|---|---|
| * *Tablet core weight (uncoated) is 700 mg. Composition is expressed based on core tablet weight* | | | | |

Eprosartan acid (600 g), Pharmatose 200M (190 g), Avicel PH102 (80 g), Starch 1500 (80 g), and Ac-Di-Sol (30 g) are screened (1000 µm) and dry-mixed. Purified water is added, followed by wet massing and drying. Magnesium stearate (10 g) and Ac-Di-Sol (10 g) are added and the resulting mixture is compressed into tablets. The tablets are coated with Opadry yellow O3B222291.

### Example 6. Preparation of a formulation containing 420 mg eprosartan acid and Effersoda-12^{™} [Reference Example]

A formulation is prepared having the composition listed in Table 2.

**Table 2**

| **Component** | **%w/w** | **mg/900 mg*** | **Function** | **Reference to Standard** |
|---|---|---|---|---|
| **Intra-granular Components** | | | | |
| Eprosartan acid | 46.7 | 420.0 | Active | Internal Monograph |
| Effer-Soda12™ | 22.2 | 200.0 | Effervescent | Ph.Eur. |
| Pharmatose DCL21 | 26.6 | 239.5 | Filler | Ph.Eur. |
| Sodium Starch Glycolate | 1.5 | 13.5 | Disintegrant | Ph.Eur. |
| Magnesium stearate | 1.0 | 9.0 | Lubricant | Ph.Eur. |

| **Extra-granular Components** | | | | |
|---|---|---|---|---|
| Sodium Starch Glycolate | 1.5 | 13.5 | Disintegrant | Ph.Eur. |
| Magnesium Stearate | 0.5 | 4.5 | Lubricant | Ph.Eur. |

| **Film Coating** | | | | |
|---|---|---|---|---|
| Opadry II Yellow 85F22122 | 4.0 | 36.0 | Coating | Internal Monograph |
| Total Tablet weight | | 936 mg | | |

| | | | | |
|---|---|---|---|---|
| **Tablet core weight (uncoated) is 900 mg. Composition is expressed based on core tablet weight* | | | | |

Eprosartan free acid (467 g), Effer-Soda-12^{™} 12 (222 g), sodium starch glycolate (15 g) and lactose monohydrate (266 g) are screened (1000 µ) and blended for 10 minutes. Magnesium stearate (10 g) is added, followed by 2 minutes blending. The resulting mixture is slugged and milled twice. Magnesium stearate (5 mg) and sodium starch glycolate (15 g) are added, followed by blending and compression into tablets. The tablets are filmcoated with Opadry I 85F22122.

### Example 7. Preparation of a formulation containing 450 mg eprosartan

A formulation is prepared on the basis of the composition outlined in Table 3 below. To this end, Eprosartan Free Acid (20.809kg), Lactose Monohydrate 200M (3.295kg), Prosolv SMCC90 (2.775kg), Starch 1500 Pregelatinised (2.775kg) and Crospovidone (Polyplasdone XL10) (0.347 kg) are screened (1000 micron) and dry mixed. Purified water is added followed by wet massing, milling and drying. Magnesium Stearate LIGA MF-2-V (1.13% w/w) and Crospovidone (Polyplasdone XL10) (1.13% w/w). are added and the resulting mixture is compressed into tablets. The tablets are coated with Opadry 03B22291.

**Table 3**

| **Component** | **% w/w per 663.8mg** | **mg/663.8 mg** | **Function** | **Reference to Standard** |
|---|---|---|---|---|
| **Intra granular** | | | | |
| Eprosartan Free Acid | 67.797 | 450.0 | Active | Internal Monograph |
| Lactose Monohydrate Pharmatose 200M | 10.734 | 71.3 | Substrate for granulation | Ph. Eur. |
| Silicified MCC ProSolv ® SMCC 90 | 9.04 | 60.0 | Substrate for granulation/ compression aid | Ph.Eur. |
| Starch Pregelatinised 1500 | 9.04 | 60.0 | Binder | Ph. Eur. |
| Crospovidone Polyplasdone XL-10 | 1.13 | 7.5 | Disintegrant | Ph.Eur. |
| Purified Water¹ | (42¹) | --- | Solvent/Diluent | Ph. Eur. |
| ***Sub Total*** | *97.74* | *648.8* | | |

| **Extra granular** | | | | |
|---|---|---|---|---|
| Crospovidone Polyplasdone XL-10 | 1.13 | 7.5 | Disintegrant | Ph. Eur. |
| Magnesium Stearate Liga MF-2-V | 1.13 | 7.5 | Lubricant | Ph. Eur. |
| **Sub Total** | **100.0** | **663.8** | | |
| Opadry 03B22291 Yellow | 3² | 19.9 | Coating Agent | Internal Monograph |
| Purified Water¹ | --- | (139.4) | Solvent/Diluent | |
| **Total** | N/A | 690.4 | | |

| | | | | |
|---|---|---|---|---|
| ¹ Removed during the process, 42% of granulation batch size ² 3 % Weight gain (Coating solution = 12.5%w/w total solids) | | | | |

### Example 8. Release profile eprosartan from different formulations

Dissolution testing from different eprosartan tablets was performed using the USP Dissolution Apparatus II with a dissolution medium 0.2 M phosphate buffer at pH 7.5, a medium volume of 1000ml with a temperature of 37 ± 0.5 °C at a paddle speed of 50 rpm, taking samples with a sample volume of 10 ml and measuring in a QS Flow cell with a path length of 1 mm at a wavelength of 235 nm. The 0.2 M phosphate buffer was prepared by dissolving 302.6 g of disodium hydrogen phosphate dihydrate and 40.8 g of potassium dihydrogen phosphate in 10 litres of pure water. The pH was adjusted to 7.50 ± 0.05 with addition of either 5 M sodium hydroxide or 85% phosphoric acid.

The dissolution profiles were as follows:

**Table 4**

| Time (min) | Eprosartan marketed 600 mg mesylate tablet (% release) | 420 mg tablet (Example 5) (% release) | 450 mg tablet (Example 7) (% release) |
|---|---|---|---|
| **0** | **0** | **0** | **0** |
| **5** | | **60** | **36** |
| **10** | **84** | | |
| **15** | **92** | **100** | **99** |
| **20** | **95** | | |
| **30** | **97** | **100** | **100** |
| **45** | **98** | **100** | **99** |
| **60** | | **100** | **100** |

### Example 9. An open-label, randomized, three-way cross-over evaluation of the relative bioavailability of two 420 mg free acid eprosartan tablets in comparison to the marketed 600 mg tablets of eprosartan In healthy adult male subjects. [Reference Example]

### Objectives:

### Primary Obiective

To assess the relative bioavailability of two experimental 420 mg eprosartan free acid tablets with the reference formulation: eprosartan 600 mg (as a mesylate) marketed tablets in healthy adult, male, human subjects under fasting conditions.

### Secondary Obiective

To assess the safety and tolerability of the eprosartan formulations.

### Methodology:

This was a single center, open-label, balanced, randomized, single dose, cross-over, comparative oral bioavailability study in 24 healthy, adult, male subjects under fasting conditions. Each subject was to participate in three study periods separated by a washout period of at least five days. Subjects received one of the following treatments in each treatment period; Treatment A (experimental 420 mg eprosartan free acid effersoda tablet according to Example 6), Treatment B (experimental 420 mg eprosartan free acid pharmatose tablet according to Example 5) and Treatment C (600 mg eprosartan marketed tablets). Subjects were screened for their eligibility to participate in the study within 28 days of their first admission. Eligible subjects were admitted to the clinic on the day prior to dosing (Day -1) and randomized to treatment. Subjects remained in the clinical unit until Day 3 of each treatment period. There was at least 5 days between dosing in adjacent treatment periods. Subjects returned to the clinical unit for a follow-up visit 5-7 days after discharge from treatment Period 3.

### Number of Subjects (Planned, Randomized and Analyzed):

24 subjects were planned, randomized and analyzed.

### Main Criteria for Inclusion:

Male subjects aged 18 to 50 years, inclusive with a body mass index (BMI) between 18 to 28 kg/m², inclusive. and body weight >50 kg or <100 kg. Subjects were to be in good health as determined by vital signs, medical history, physical examination, serum biochemistry, urinalysis and hematology.

### Test Product, Dose and Mode of Administration, Batch Number:

Treatment A: 420 mg eprosartan free acid effersoda oral tablet prepared in accordance with Example 6.
Treatment B: 420 mg eprosartan free acid pharmatose oral tablet, prepared in accordance with Example 5.

### Duration of Treatment:

Subjects received a single dose of Treatment A, B and C in three treatment periods. There was at least 5 days between dosing in adjacent periods.

### Reference Therapy, Dose and Mode of Administration, Batch Number:

Treatment C: 600 mg eprosartan marketed tablets,

### Criteria for Evaluation

### Pharmacokinetics:

The following pharmacokinetic (PK) parameters for eprosartan were determined: λ_{z}, AUC, AUC/D, AUC₀₋ₜ, AUC₀₋ₜ/D, Cₘₐₓ, Cₘₐₓ/D, CUF, t_{1/2}, tₘₐₓ, V_{z}/F and bioavailability parameters (F_{AUC}%, F_{AUC(0-t)}%, F_{Cmax}%, F_{AUC/D}%, F_{AUC(0-t)/D}%, F_{Cmax}/D%). The AUC, AUC₀₋ₜ, and Cₘₐₓ were considered as the primary PK parameters.

### Statistical Methods:

### Pharmacokinetics:

Plasma concentrations of eprosartan were summarized by treatment and nominal measurement time using descriptive statistics. Concentrations below the LLOQ were set to ½ LLOQ prior to calculation of descriptive statistics. Descriptive statistics were only calculated if at least 2/3 of the data were ≥ LLOQ. Eprosartan pharmacokinetic parameters were summarized by treatment using descriptive statistics.

A mixed model analysis of variance (ANOVA) of the primary pharmacokinetic parameters Cₘₐₓ, AUC₀₋ₜ, and AUC was used to compare the relative bioavailability of the test formulations (experimental 420 mg free acid tablets) with the reference formulation (eprosartan 600 mg tablet). A model with fixed effect terms for period, sequence and treatment and subject within sequence as random effect was used. The pharmacokinetic parameters were log transformed before analysis.

From this ANOVA, least-squares means for each treatment, estimated treatment differences, and 90% confidence intervals for treatment differences were calculated. The log-transformed results were transformed to the original scale by exponentiation to obtain geometric least squares means, treatment ratios and their 90% confidence intervals.

Similar relative bioavailability comparisons between the test and the reference formulations were performed on dose-normalized parameters (Cₘₐₓ/D, AUC₀₋ₜ/D, and AUC/D).

### Summary - Conclusions

### Pharmacokinetic Results:

Samples were analyzed for concentrations of eprosartan using validated high performance liquid chromatography with tandem mass spectrometry detection (lower limit of quantitation: 1 ng/mL).

Following administration of the eprosartan effersoda tablet (Trt A, test formulation), pharmatose tablet (Trt B, test formulation) or the marketed tablet (Trt C, reference formulation), the plasma concentrations decreased in a mostly biphasic manner and were quantifiable for up to 48 hours post administration (last time point evaluated). The concentration time profiles for eprosartan for the three treatments were similar at later time points (>10 h) but there were differences in the profiles in the first 10 hours following dosing (ie, absorption phase). The effersoda formulation showed a more rapid absorption and a higher Cₘₐₓ, while the pharmatose and the marketed formulations showed similar absorption profiles.

The summary of key PK parameters for eprosartan is presented in Table 5:

Based on non-normalized eprosartan parameters, mean overall exposure (AUC and AUC₀₋ₜ) for the effersoda tablet was 7-12% higher and mean Cₘₐₓ was 34% higher compared to the marketed tablet. In the case of the pharmatose tablet, AUC and Cₘₐₓ were similar to the marketed tablet (<5% difference), but the mean AUC₀₋ₜ was 11 % lower (see table 4).

Based on dose-normalized eprosartan parameters, mean overall exposure (AUC and AUC₀₋ₜ) for the effersoda tablet was 52-60% higher and mean Cₘₐₓ was 92% higher compared to the marketed tablet. Similarly, in the case of the pharmatose tablet, mean overall exposure (AUC and AUC₀₋ₜ) was 28-47% higher and mean Cₘₐₓ was 38% higher compared to the marketed tablet (see table 4).

**Table 5**

| Summary of Key Non-Normalized and Dose-Normalized Eprosartan Plasma Pharmacokinetic Parameters | | | | | | |
|---|---|---|---|---|---|---|
| | Treatment A | | Treatment B | | Treatment C | |
| Parameter | N | Mean (SD) | N | Mean (SD) | N | Mean (SD) |
| AUC (ng·h/mL) | 18 | 10020 (4969) | 18 | 9277 (4644) | 19 | 7695 (3846) |
| DN- AUC | 18 | 23.86 (11.81) | 18 | 22.08 (11.05) | 19 | 12.81 (6.39) |
| AUC₀₋ₜ(ng·h/mL) | 24 | 9470 (5173) | 23 | 8139 (4519) | 24 | 9415 (7268) |
| DN-AUC0-t | 24 | 22.53 (12.3) | 23 | 19.39 (10.78) | 24 | 15.70 (12.13) |
| Cₘₐₓ (ng/mL) | 24 | 3425 (1911) | 23 | 2533 (1541) | 24 | 2652(1947) |
| DN-Cmax | 24 | 8.153 (4.547) | 23 | 6.030 (3.668) | 24 | 4.420 (3.242) |
| tₘₐₓ (h)^{[a]} | 24 | 0.52 (1.25-4.00) | 23 | 2.00 (0.75-4.03) | 24 | 1.00 (1.50-3.50) |
| T_{1/2} (h) | 18 | 10.47 (3.04) | 18 | 15.6 (10.85) | 19 | 16.14 (9.11) |
| Vz/F(L) | 18 | 868.8 (646.2) | 18 | 1323 (1178) | 19 | 2398(1662) |
| CUF (Uh) | 18 | 53.97 (28.64) | 18 | 58.76 (37.99) | 19 | 96.86 (44.05) |

| | | | | | | |
|---|---|---|---|---|---|---|
| SD=standard deviation ; DN= Dose-Normalized Treatment A: Eprosartan free acid (420 mg) effersoda tablet; Treatment B: Eprosartan free acid (420 mg) pharmatose tablet; Treatment C: Eprosartan (600 mg) marketed tablet [a] Median (range) | | | | | | |

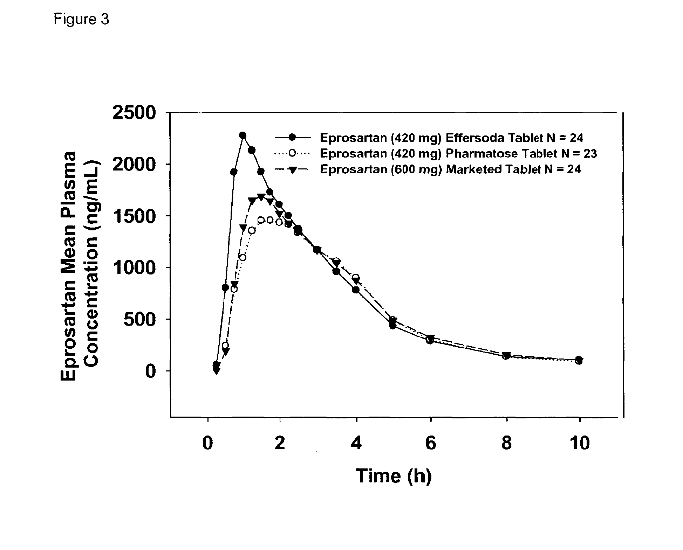

Following single dose oral administration of the eprosartan (420 mg) effersoda formulation, eprosartan (420 mg) pharmatose formulation, or the eprosartan (600 mg) marketed formulation, there was a rapid increase in the plasma concentrations of eprosartan up to approximately 0.5 to 2 hours (median tₘₐₓ), followed by a biphasic decline in plasma concentrations. Geometric mean plasma concentrations of eprosartan were quantifiable in plasma for up to 48 hours (last time point for PK evaluation) following oral administration of all the three formulations. The concentration time profiles of eprosartan for the three treatments were similar at later time points (>10 h) but there were differences in the profiles in the first 10 hours following dosing (ie, absorption phase). The geometric mean eprosartan plasma concentration time profiles for these first 10 hours are shown in Figure 3. The effersoda formulation showed a more rapid absorption and a higher Cₘₐₓ, while the pharmatose and the marketed formulations showed similar absorption profiles.

### Conclusion:

### Pharmacokinetic Conclusions

- The relative bioavailability of eprosartan from the effersoda formulation compared to the marketed formulation (unadjusted for dose) is 112% [90% Cl: 96 -127 %] (AUC), 107% [90% Cl: 93 -123 %] (AUC₀₋ₜ), and 134% [90% Cl: 113 -159 %] (Cₘₐₓ).
- The relative bioavailability of eprosartan from the pharmatose formulation compared to the marketed formulation (unadjusted for dose) is 103% [90% Cl: 88 -120 %] (AUC), 89% [90% Cl: 78 -103 %] (AUC₀₋ₜ), and 96% [90% Cl: 81 -114 %] (Cₘₐₓ). The pharmatose formulation provides a similar eprosartan exposure as the marketed formulation.
- When adjusted for dose differences, there is a 60% [90% Cl: 38 -85 %] and 52% [90% Cl: 33 -75 %] increase in eprosartan AUC and AUC₀₋ₜ, and an approximately 92% [90% Cl: 62 -127 %] increase in Cₘₐₓ with the effersoda formulation as compared to the marketed formulation.
- When adjusted for dose differences, there is a 47% [90% Cl: 26 -71 %] and 28% [90% Cl: 11 -47 %] increase in eprosartan AUC and AUC₀₋ₜ, and an approximately 38% [90% Cl: 16 -63 %] increase in Cₘₐₓ with the pharmatose formulation as compared to the marketed formulation.

As to safety, both the effersoda and pharmatose test formulations of eprosartan were well tolerated by the healthy subjects in the study.

## Claims

1. Eprosartan acid for use in a method of treating a disorder modulated by blocking angiotensin II (AII) receptors selected from the group consisting of hypertension, congestive heart failure, renal failure, and combinations thereof, comprising the step of administering to a subject in need thereof a daily dose of 440-460 mg of said eprosartan acid, wherein said daily dose of eprosartan acid is administered in a single, immediate release oral formulation.

2. Eprosartan acid for use in a method of treatment according to claim 1, comprising the daily administration of 450 mg of eprosartan acid.

3. Eprosartan acid for use in a method of treatment according to claim 1 or 2, wherein the eprosartan acid is administered in a pharmaceutical formulation exhibiting a release of eprosartan acid of at least 95% in 15 minutes, preferably exhibiting a release profile of: at least 30% in 5 minutes, at least 95% in 15 minutes, and 100% in 30 minutes, the release being determined in accordance with USP by means of dissolution testing using USP Dissolution Apparatus II with a dissolution medium 0.2 M phosphate buffer at pH 7.5, a medium volume of 1000ml with a temperature of 37 ± 0.5 °C at a paddle speed of 50 rpm, taking samples with a sample volume of 10 ml and measuring in a QS Flow cell with a path length of 1 mm at a wavelength of 235 nm, with the 0.2 M phosphate buffer being prepared by dissolving 302.6 g of disodium hydrogen phosphate dihydrate and 40.8 g of potassium dihydrogen phosphate in 10 litres of pure water and pH adjusted to 7.50 ± 0.05 with addition of either 5 M sodium hydroxide or 85% phosphoric acid.

4. An immediate release oral pharmaceutical formulation comprising 440-460 mg of eprosartan acid.

5. A pharmaceutical formulation according to claim 4, comprising 450 mg of eprosartan acid.

6. A pharmaceutical formulation according to claim 4 or 5, exhibiting a release of eprosartan acid of at least 95% in 15 minutes, preferably exhibiting a release profile of: at least 30% in 5 minutes, at least 95% in 15 minutes, and 100% in 30 minutes, the release being determined in accordance with USP by means of dissolution testing using USP Dissolution Apparatus II with a dissolution medium 0.2 M phosphate buffer at pH 7.5, a medium volume of 1000ml with a temperature of 37 ± 0.5 °C at a paddle speed of 50 rpm, taking samples with a sample volume of 10 ml and measuring in a QS Flow cell with a path length of 1 mm at a wavelength of 235 nm, with the 0.2 M phosphate buffer being prepared by dissolving 302.6 g of disodium hydrogen phosphate dihydrate and 40.8 g of potassium dihydrogen phosphate in 10 liters of pure water and pH adjusted to 7.50 ± 0.05 with addition of either 5 M sodium hydroxide or 85% phosphoric acid.

7. A pharmaceutical formulation according to any one of the claims 4-6, comprising alpha lactose monohydrate as a pharmaceutically acceptable excipient, preferably alpha lactose 200M.

8. A pharmaceutical formulation according to claim 7, further comprising cross-linked N-vinyl-2-pyrrolidone.

9. A pharmaceutical formulation according to claim 8, comprising dry-mix granules of lactose monohydrate 200M and microcrystalline cellulose, more preferably silicified microcrystalline cellulose, starch and cross-linked N-vinyl-2-pyrrolidone.

10. An immediate release oral pharmaceutical formulation, in the form of a dosage unit comprising 450 mg of eprosartan acid, 71.25 mg of alpha lactose monohydrate 200M, 60.0 mg of silicified microcrystalline cellulose, 9.040 mg of starch, 15 mg of cross-linked N-vinyl-2-pyrrolidone and 7.5 mg of magnesium stearate.

11. A pharmaceutical formulation according to any one of the claims 4-10, comprising a diuretic compound, preferably hydrochlorothiazide, as further active ingredient.

12. A pharmaceutical formulation according to any one of the claims 4-11, for use in a method of treatment of a disorder modulated by blocking angiotensin II (AII) receptors selected from the group consisting of hypertension, congestive heart failure, renal failure, and combinations thereof, by daily administering to a subject in need thereof, eprosartan acid in a daily dose within the range of 440-460 mg.

13. A pharmaceutical formulation for use according to claim 12, wherein the daily dose of eprosartan acid is 450 mg.

14. Use of eprosartan acid for the manufacture of a medicament for the treatment of a disorder modulated by blocking angiotensin II (AII) receptors selected from the group consisting of hypertension, congestive heart failure, renal failure, and combinations thereof, comprising the step of administering to a subject in need thereof a daily dose of 440-460 mg of eprosartan acid, wherein said daily dose of eprosartan acid is administered in a single, immediate release oral formulation.

15. The use according to claim 14, comprising the daily administration of 450 mg of eprosartan acid.

## Patentansprüche

1. Eprosartansäure zur Verwendung in einem Verfahren zur Behandlung einer Krankheit, moduliert durch Blockieren von Angiotensin II (AII)-Rezeptoren, ausgewählt aus der Gruppe bestehend aus Bluthochdruck, kongestiver Herzinsuffizienz, Niereninsuffizienz und Kombinationen davon, umfassend den Schritt, bei dem einem Patienten, der dies benötigt, eine Tagesdosis von 440-460 mg der Eprosartansäure verabreicht wird, wobei die Tagesdosis an Eprosartansäure in einer einzelnen, sofort freigesetzten oralen Formulierung verabreicht wird.

2. Eprosartansäure zur Verwendung in einem vVerfahren zur Behandlung nach Anspruch 1, umfassend die tägliche Verabreichung von 450 mg Eprosartansäure.

3. Eprosartansäure zur Verwendung in einem Verfahren zur Behandlung nach Anspruch 1 oder 2, wobei die Eprosartansäure in einer pharmazeutischen Rezeptur mit einer Freisetzung von Eprosartansäure von mindestens 95 % in 15 Minuten, bevorzugt mit einem Freisetzungsprofil von: mindestens 30 % in 5 Minuten, mindestens 95 % in 15 Minuten und 100 % in 30 Minuten, verabreicht wird, wobei die Freisetzung gemäß USP (U.S. Pharmacopeial Convention)-Referenznormen mittels Auflösungstests unter Verwendung eines USP Auflösungsapparats II mit einem Auflösungsmedium 0,2 M Phosphatpuffer bei 7,5 pH, einem Mediumvolumen von 1000 ml mit einer Temperatur von 37 ± 0,5 °C bei einer Paddelgeschwindigkeit von 50 U/Min. ermittelt wird, wobei die Proben mit einem Probenvolumen von 10 ml entnommen und in einer QS-Flusszelle mit einer Weglänge von 1 mm bei einer Wellenlänge von 235 nm gemessen werden, wobei der 0,2 M Phosphatpuffer durch Auflösen von 302,6 g Dinatriumhydrogenphosphatdihydrat und 40,8 g Kaliumdihydrogenphosphat in 10 l reinem Wasser und einem pH, eingestellt auf 7,50 ± 0,05, unter Hinzufügung von entweder 5 M Natriumhydroxid oder 85 % Phosphorsäure hergestellt wird.

4. Orale pharmazeutische Formulierung zur sofortigen Freisetzung, umfassend 440-460 mg Eprosartansäure.

5. Pharmazeutische Formulierung nach Anspruch 4, umfassend 450 mg Eprosartansäure.

6. Pharmazeutische Formulierung nach Anspruch 4 oder 5, mit einer Freisetzung von Eprosartansäure von mindestens 95 % in 15 Minuten, bevorzugt mit einem Freisetzungsprofil von: mindestens 30 % in 5 Minuten, mindestens 95 % in 15 Minuten und 100 % in 30 Minuten, verabreicht wird, wobei die Freisetzung gemäß USP (U.S. Pharmacopeial Convention)-Referenznormen mittels Auflösungstests unter Verwendung eines USP Auflösungsapparats II mit einem Auflösungsmedium 0,2 M Phosphatpuffer bei 7,5 pH, einem Mediumvolumen von 1000 ml mit einer Temperatur von 37 ± 0,5 °C bei einer Paddelgeschwindigkeit von 50 U/Min. ermittelt wird, wobei die Proben mit einem Probenvolumen von 10 ml genommen und in einer QS-Flusszelle mit einer Weglänge von 1 mm bei einer Wellenlänge von 235 nm gemessen werden, wobei der 0,2 M Phosphatpuffer durch Auflösen von 302,6 g Dinatriumhydrogenphosphatdihydrat und 40,8 g Kaliumdihdrogenphosphat in 10 l reinem Wasser und einem pH, eingestellt auf 7,50 ± 0,05, unter Hinzufügung von entweder 5 M Natriumhydroxid oder 85 % Phosphorsäure hergestellt wird.

7. Pharmazeutische Formulierung nach einem der Ansprüche 4-6, umfassend Alphalactosemonohydrat als pharmazeutisch akzeptablen Hilfsstoff, bevorzugt Alphalactose 200M.

8. Pharmazeutische Formulierung nach Anspruch 7, ferner umfassend quervernetztes N-Vinyl-2-pyrrolidon.

9. Pharmazeutische Formulierung nach Anspruch 8, umfassend Trockenmischgranulat aus Lactosemonohydrat 200M und mikrokristalline Zellulose, bevorzugter silizifizierte mikrokristalline Zellulose, Stärke und quervernetztes N-Vinyl-2-pyrrolidon.

10. Orale pharmazeutische Formulierung zur sofortigen Freisetzung in Form einer Dosierungseinheit, umfassend 450 mg Eprosartansäure, 71,25 mg Alphalactosemonohydrat 200M, 60,0 mg silifizierte mikrokristalline Zellulose, 9,040 mg Stärke, 15 mg quervernetztes N-Vinyl-2-pyrrolidon und 7,5 mg Magnesiumstearat.

11. Pharmazeutische Formulierung nach einem der Ansprüche 4-10, umfassend eine diuretische Verbindung, bevorzugt Hydrochlorthiazid, als weiteren aktiven Bestandteil.

12. Pharmazeutische Formulierung nach einem der Ansprüche 4-11 zur Verwendung in einem Verfahren zur Behandlung einer Krankheit, moduliert durch Blockieren von Angiotensin II (AII)-Rezeptoren, ausgewählt aus der Gruppe bestehend aus Bluthochdruck, kongestiver Herzinsuffizienz, Niereninsuffizienz und Kombinationen davon, durch tägliche Verabreichung von Eprosartansäure in einer Tagesdosis im Bereich von 440-460 mg an einen Patienten, der dies benötigt.

13. Pharmazeutische Formulierung zur Verwendung nach Anspruch 12, wobei die Tagesdosis an Eprosartansäure 450 mg beträgt.

14. Verwendung von Eprosartansäure zur Herstellung eines Arzneimittels für die Behandlung einer Krankheit, moduliert durch Blockieren von Angiotensin II (AII)-Rezeptoren, ausgewählt aus der Gruppe bestehend aus Bluthochdruck, kongestiver Herzinsuffizienz, Niereninsuffizienz und Kombinationen davon, umfassend den Schritt, bei dem einem Patienten, der dies benötigt, eine Tagesdosis von 440-460 mg der Eprosartansäure verabreicht wird, wobei die Tagesdosis an Eprosartansäure in einer einzelnen, sofort freigesetzten oralen Rezeptur verabreicht wird.

15. Verwendung nach Anspruch 14, umfassend die tägliche Verabreichung von 450 mg Eprosartansäure.

## Revendications

1. Acide éprosartan destiné à être utilisé dans un procédé de traitement d'un trouble modulé par le blocage des récepteurs d'angiotensine II (AII) choisi dans le groupe consistant en l'hypertension, l'insuffisance cardiaque congestive, l'insuffisance rénale et leurs combinaisons, comprenant l'étape d'administration à un sujet qui en a besoin d'une dose journalière de 440-460 mg dudit acide éprosartan, où ladite dose journalière d'acide éprosartan est administrée dans une seule formulation orale à libération immédiate.

2. Acide éprosartan destiné à être utilisé dans un procédé de traitement selon la revendication 1, comprenant l'administration journalière de 450 mg d'acide éprosartan.

3. Acide éprosartan destiné à être utilisé dans un procédé de traitement selon la revendication 1 ou 2, où l'acide éprosartan est administré dans une formulation pharmaceutique présentant une libération d'acide éprosartan d'au moins 95 % en 15 minutes, de préférence présentant un profil de libération de : au moins 30 % en 5 minutes, au moins 95 % en 15 minutes et 100 % en 30 minutes, la libération étant déterminée selon USP au moyen d'un test de dissolution utilisant l'appareil de dissolution USP II avec un milieu de dissolution tampon phosphate 0,2 M à pH 7,5, un volume de milieu de 1 000 ml avec une température de 37 ± 0,5°C à une vitesse de palette de 50 tr/min, en prélevant des échantillons avec un volume d'échantillon de 10 ml et en mesurant dans une cuve à circulation QS avec une longueur de passage de 1 mm à une longueur d'onde de 235 nm, le tampon phosphate 0,2 M étant préparé par dissolution de 302,6 g d'hydrogénophosphate de disodium dihydraté et de 40,8 g de dihydrogénophosphate de potassium dans 10 litres d'eau pure et le pH ajusté à 7,50 ± 0,05 par addition d'hydroxyde de sodium 5 M ou d'acide phosphorique à 85 %.

4. Formulation pharmaceutique orale à libération immédiate comprenant 440-460 mg d'acide éprosartan.

5. Formulation pharmaceutique selon la revendication 4, comprenant 450 mg d'acide éprosartan.

6. Formulation pharmaceutique selon la revendication 4 ou 5, présentant une libération d'acide éprosartan d'au moins 95 % en 15 minutes, de préférence présentant un profil de libération de : au moins 30 % en 5 minutes, au moins 95 % en 15 minutes et 100 % en 30 minutes, la libération étant déterminée selon USP au moyen d'un test de dissolution utilisant l'appareil de dissolution USP II avec un milieu de dissolution tampon phosphate 0,2 M à pH 7,5, un volume de milieu de 1 000 ml avec une température de 37 ± 0,5°C à une vitesse de palette de 50 tr/min, en prélevant des échantillons avec un volume d'échantillon de 10 ml et en mesurant dans une cuve à circulation QS avec une longueur de passage de 1 mm à une longueur d'onde de 235 nm, le tampon phosphate 0,2 M étant préparé par dissolution de 302,6 g d'hydrogéno-phosphate de disodium dihydraté et de 40,8 g de dihydrogénophosphate de potassium dans 10 litres d'eau pure et le pH ajusté à 7,50 ± 0,05 par addition d'hydroxyde de sodium 5 M ou d'acide phosphorique à 85 %.

7. Formulation pharmaceutique selon l'une quelconque des revendications 4-6, comprenant du lactose alpha monohydraté comme excipient pharmaceutiquement acceptable, de préférence du lactose alpha 200M.

8. Formulation pharmaceutique selon la revendication 7, comprenant en outre de la N-vinyl-2-pyrrolidone réticulée.

9. Formulation pharmaceutique selon la revendication 8, comprenant des granules mélangés à sec de lactose monohydraté 200M et de cellulose microcristalline, de préférence encore de cellulose microcristalline silicifiée, d'amidon et de N-vinyl-2-pyrrolidone réticulée.

10. Formulation pharmaceutique orale à libération immédiate sous forme d'une dose unitaire comprenant 450 mg d'acide éprosartan, 71,25 mg de lactose alpha monohydraté 200M, 60,0 mg de cellulose microcristalline silicifiée, 9,040 mg d'amidon, 15 mg de N-vinyl-2-pyrrolidone réticulée et 7,5 mg de stéarate de magnésium.

11. Formulation pharmaceutique selon l'une quelconque des revendications 4-10, comprenant un composé diurétique, de préférence l'hydrochlorothiazide, comme autre ingrédient actif.

12. Formulation pharmaceutique selon l'une quelconque des revendications 4-11, destinée à être utilisée dans un procédé de traitement d'un trouble modulé par le blocage des récepteurs d'angiotensine II (AII) choisi dans le groupe consistant en l'hypertension, l'insuffisance cardiaque congestive, l'insuffisance rénale et leurs combinaisons, par administration journalière à un sujet qui en a besoin d'acide éprosartan en une dose journalière dans la plage de 400-460 mg.

13. Formulation pharmaceutique destinée à être utilisée selon la revendication 12, où la dose journalière d'acide éprosartan est 450 mg.

14. Utilisation d'acide éprosartan pour la fabrication d'un médicament pour le traitement d'un trouble modulé par le blocage des récepteurs d'angiotensine II (AII) choisi dans le groupe consistant en l'hypertension, l'insuffisance cardiaque congestive, l'insuffisance rénale et leurs combinaisons, comprenant l'étape d'administration à un sujet qui en a besoin d'une dose journalière de 440-460 mg d'acide éprosartan, où ladite dose journalière d'acide éprosartan est administrée dans une seule formulation orale à libération immédiate.

15. Utilisation selon la revendication 14, comprenant l'administration journalière de 450 mg d'acide éprosartan.
